# EUROPEAN PATENT APPLICATION

(11) **EP 4 740 912 A1**
(43) Date of publication of application: **13.05.2026**
(21) Application number: 24836291.5
(22) Date of filing: 02.07.2024
(51) Int. Cl.: A61F 2/44, A61F 2/30

(54) **INTERVERTEBRAL BODY FUSION PROSTHESIS**

(30) Priority: 03.07.2023 KR 20230085878; 01.07.2024 KR 20240086227
(71) Applicant: CG Bio Co., Ltd., Seoul 04349 (KR); INNOTEAM.INC, Seoul 06149 (KR)
(72) Inventor: JUNG, Ui Su, Seoul 05393 (KR); CHO, Samuel, Englewood Cliffs,, New Jersey 07632 (US)
(74) Representative: Laine IP Oy
(86) International application number: PCT/KR2024/009279
(87) International publication number: WO 2025/009843

(57) **Abstract**

An intervertebral fusion cage is provided. The intervertebral fusion cage according to an aspect of the present invention comprises: a screw including a screw head and a screw body which extends from the screw head in a first direction and has a screw thread on an outer circumferential surface thereof; a main body including a screw housing in which the screw head is rotatably accommodated and which has a through-portion penetrating in a second direction inclined with respect to the first direction, and a base plate which is disposed on one surface of the screw housing at the second-direction side and has a base hole in communication with the through-portion; a guide member including a guide body in which the screw housing is accommodated and to which the screw body is rotatably coupled, the guide member being movable relative to the main body in a direction parallel to the first direction according to rotation of the screw; a moving plate movably connected to the main body and displaced according to relative movement between the main body and the guide member; and at least one bar-type member disposed across the through-portion.

## Description

### TECHNICAL FIELD

The present invention relates to an intervertebral fusion cage, and more particularly, to an intervertebral fusion cage applicable to an implant requiring in-body expansion.

### BACKGROUND

An intervertebral fusion cage is a device used in spinal fusion, which is a surgical treatment method for spinal disorders. When a disc ruptures or weakens due to a disease or accident, the spinal nerve may be compressed, causing pain. In such a case, the damaged disc is removed, and spinal fusion is performed by inserting an intervertebral fusion cage into the intervertebral space from which the damaged disc has been removed, to restore and maintain the intervertebral spacing and lordosis.

As described above, the intervertebral fusion cage is inserted into the intervertebral space between vertebral bodies from which a degenerative disc has been removed, thereby providing a space for bone growth required for fusion. In addition, the intervertebral fusion cage alleviates pain by securing an appropriate spacing between the vertebral bodies and restores spinal stability.

Generally, intervertebral fusion cages are individually manufactured according to the lordosis and height of the spine. However, during surgery performed through a posterior approach to the spine, it is often difficult to use an intervertebral fusion cage having the lordosis and height required for a patient. This is because most patients who require spinal fusion experience degenerative changes in the spine, making it difficult to secure sufficient space for inserting the intervertebral fusion cage.

In this situation, expandable intervertebral fusion cages have been introduced, which have a low height at the time of implantation to allow easy insertion into the disc space and can expand the lordosis or height after being positioned at a desired location. However, most of the conventional expandable intervertebral fusion cages are configured to expand only one of the lordosis or the height, rather than both.

In conventional cases, to promote bone formation, a bone graft material was filled after the installation of the intervertebral fusion cage. However, such a bone graft material focused on promoting bone growth and did not contribute to structural stability against external forces.

After implantation, the intervertebral fusion cage is used long-term in an expanded state as the patient's bone grows inside it. Since the process of bone formation is guided by the position and shape of the bone graft material and the intervertebral fusion cage, it is very important to secure structural stability so that the implanted intervertebral fusion cage can maintain its rigidity during long-term use and throughout the bone formation process.

Accordingly, there is a growing need to develop an intervertebral fusion cage that can adjust both the lordosis and the height, operate stably, and maintain a rigid structure during the process of bone formation and maintenance while also promoting bone growth.

### DISCLOSURE

### TECHNICAL PROBLEM

The present invention has been made to solve the problems described above, and an object of the present invention is to provide an intervertebral fusion cage capable of adjusting both the lordosis and the height through a driving mechanism that converts motion between nonlinear and linear movements.

Another object of the present invention is to provide an intervertebral fusion cage capable of maintaining a rigid structure even after implantation, expansion, and fixation.

Still another object of the present invention is to provide an intervertebral fusion cage that can enhance the efficiency of bone formation and fusion while maintaining structural rigidity.

The technical problems of the present invention are not limited to those described above, and other problems not mentioned herein will be clearly understood by those skilled in the art from the following description.

### TECHNICAL SOLUTION

According to an aspect of the present invention, provided is an intervertebral fusion cage, comprising: a screw including a screw head and a screw body which extends from the screw head in a first direction and has a screw thread on an outer circumferential surface of the screw body; a main body including a screw housing in which the screw head is rotatably accommodated and which has a through-portion penetrating in a second direction inclined with respect to the first direction, and a base plate which is disposed on one surface of the screw housing on the second-direction side and has a base hole in communication with the through-portion; a guide member including a guide body in which the screw housing is accommodated and to which the screw body is rotatably coupled, the guide member being movable relative to the main body in a direction parallel to the first direction according to rotation of the screw; a moving plate movably connected to the main body and displaced according to relative movement between the main body and the guide member; and at least one bar-type member disposed across the through-portion.

In this case, the bar-type member may be disposed at an end of the through-portion on the second-direction side.

In this case, the bar-type member may be disposed to extend across the through-portion along the first direction.

In this case, a plurality of the bar-type members may be arranged in parallel with each other.

In this case, a plurality of the bar-type members may be spaced apart from each other and disposed at positions having different heights.

In this case, a plurality of the bar-type members may be provided, and at least two of the bar-type members may be disposed such that at least a portion thereof overlap with each other when viewed in the second direction.

In this case, the base plate may include at least one resistance protrusion formed to protrude on the one surface on the second-direction side, and the resistance protrusion may extend in a third direction perpendicular to the first direction.

In this case, a plurality of the resistance protrusions may be formed, at least one of the resistance protrusions may be formed on a portion of the one surface of the base plate on the second-direction side where the base hole is not located, and at least one of the resistance protrusions may be formed such that an end portion of the resistance protrusion extends across the base hole along the third direction.

In this case, a porous member made of a porous material may be provided in the base hole.

In this case, the porous member may be provided in a shape corresponding to the shape of the base hole.

In this case, the second direction may be formed at an angle perpendicular to the first direction, and an area of a cross section obtained by cutting the base hole in a direction perpendicular to the second direction may be larger than an area of a cross section obtained by cutting an end portion of the through-portion on the second-direction side in the direction perpendicular to the second direction.

In this case, the moving plate may include at least one upper resistance protrusion formed to protrude on one surface thereof farthest from the base plate, and the upper resistance protrusion may extend in a third direction perpendicular to the first direction.

### ADVANTAGEOUS EFFECT

According to the above configuration, in an aspect of the present invention, the intervertebral fusion cage allows both the lordosis and the height of the spine to be adjusted through a plate that first elevates a front portion thereof in a vertical direction to form a predetermined angle according to the rotation of the screw and then moves vertically as a whole to increase its height, thereby enabling an appropriate lordosis and height of the spine to be secured for each patient during spinal fusion.

In another aspect of the present invention, the intervertebral fusion cage can effectively resist external forces applied from different directions by reinforcing the interior with bar-type members extending in a direction perpendicular to the extending direction of protrusions formed on a contact surface of the plate, thereby maintaining structural rigidity.

In yet another aspect of the present invention, the intervertebral fusion cage can further enhance structural stability by forming different cross-sectional areas of the base hole and the through-portion along the bone growth path.

In still another aspect of the present invention, the intervertebral fusion cage can form an efficient multi-stage structure in which a porous member and a bar-type member are sequentially arranged along the bone growth path, thereby promoting bone fusion in an initial stage and, after bone fusion is facilitated, securing additional bone fusion promotion together with structural stability.

Advantageous effects of the present invention are not limited to the above-described effects, and should be understood to include all effects that can be inferred from the configuration of the invention described in the detailed description or claims of the present invention.

### BRIEF DESCRIPTION OF THE DRAWINGS

FIG. 1 is a rear perspective view of an intervertebral fusion cage according to an exemplary embodiment of the present invention.
FIG. 2 is a bottom perspective view of an intervertebral fusion cage according to an exemplary embodiment of the present invention.
FIG. 3 is an exploded perspective view of an intervertebral fusion cage according to an exemplary embodiment of the present invention.
FIG. 4 is a top view of an intervertebral fusion cage according to an exemplary embodiment of the present invention.
FIG. 5 is a cross-sectional view showing a section of the intervertebral fusion cage taken along line I-I' of FIG. 4.
FIG. 6 is a cross-sectional view showing a section of the intervertebral fusion cage taken along line II-II' of FIG. 4.
FIG. 7 is a cross-sectional view of an intervertebral fusion cage according to an exemplary embodiment of the present invention, including a section cut in a direction perpendicular to the direction of a screw body at the position of a screw head.
FIG. 8 is a rear perspective view showing an intervertebral fusion cage in an expanded state of lordosis according to an exemplary embodiment of the present invention.
FIG. 9 is a left side view of the intervertebral fusion cage shown in FIG. 8.
FIG. 10 is a side cross-sectional view illustrating a process in which an angle between a front guide unit and a front guided unit of the intervertebral fusion cage according to an exemplary embodiment of the present invention is aligned.
FIG. 11 is a perspective view showing an intervertebral fusion cage in a state in which the height of a plate is increased after expansion of the lordosis according to an exemplary embodiment of the present invention.
FIG. 12 is a left side view of the intervertebral fusion cage shown in FIG. 11.
FIG. 13 is a view illustrating a process in which the intervertebral fusion cage according to an exemplary embodiment of the present invention is used in a PLIF (Posterior Lumbar Interbody Fusion).
FIG. 14 is a view illustrating a process in which the intervertebral fusion cage according to an exemplary embodiment of the present invention is used in a TLIF (Transforaminal Lumbar Interbody Fusion).

### MODES OF THE INVENTION

Hereinafter, exemplary embodiments of the present invention will be described in detail so that those of ordinary skill in the art can readily implement the present invention with reference to the accompanying drawings. The present invention may be embodied in many different forms and is not limited to the embodiments set forth herein. In the drawings, portions irrelevant to the description have been omitted for clarity of explanation of the present invention, and throughout the specification, the same reference numerals are used to denote identical or similar components.

The words and terms used in the present specification and claims should not be interpreted as being limited to their ordinary or dictionary meanings, but should be construed as having meanings and concepts consistent with the technical spirit of the present invention, in accordance with the principle that an inventor may define terms and concepts to best describe their invention.

It should be understood that the terms "comprise or include" or "have" or the like when used in this specification, are intended to describe the presence of stated features, numbers, steps, operations, elements, components and/or a combination thereof but not preclude the possibility of the presence or addition of one or more other features, numbers, steps, operations, elements, components, or a combination thereof.

In the drawings, the thicknesses and sizes of components are exaggerated to clearly illustrate the features of the structures, and the thicknesses or sizes shown in the drawings are not necessarily depicted to scale.

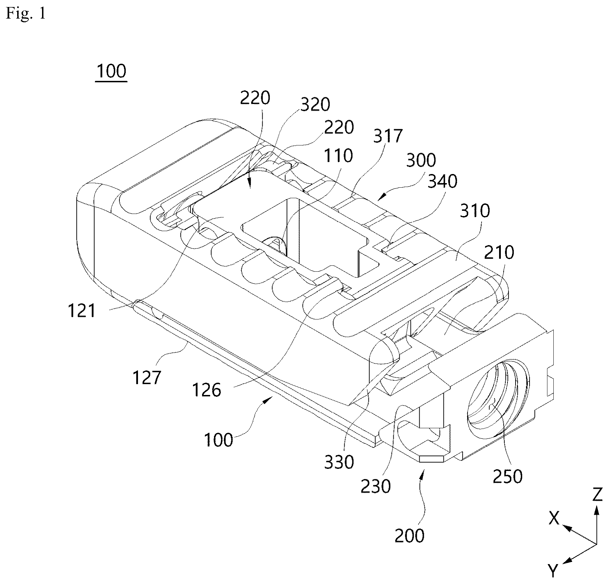

In the following description of the drawings, each direction is defined with reference to the coordinate axes shown in FIG. 1. More specifically, the positive direction of the z-axis is defined as the upper side, and the negative direction of the z-axis is defined as the lower side. The positive direction of the y-axis is defined as the left side, and the negative direction of the y-axis is defined as the right side. The positive direction of the x-axis is defined as the front side, and the negative direction of the x-axis is defined as the rear side.

In the following description, explanations of some components may be omitted to clarify the features of the present invention.

An intervertebral fusion cage 1 according to an exemplary embodiment of the present invention is a device used in spinal fusion, which is a surgical treatment method for spinal disorders. The intervertebral fusion cage 1 according to an exemplary embodiment of the present invention includes a main body 100 as a driving system. In addition, the intervertebral fusion cage 1 according to an exemplary embodiment of the present invention is inserted between vertebral bodies from which a degenerative disc has been removed and serves to support the space between the vertebral bodies until spinal fusion is achieved.

The intervertebral fusion cage 1 according to an exemplary embodiment of the present invention enables both the lordosis and the height of the spine to be secured.

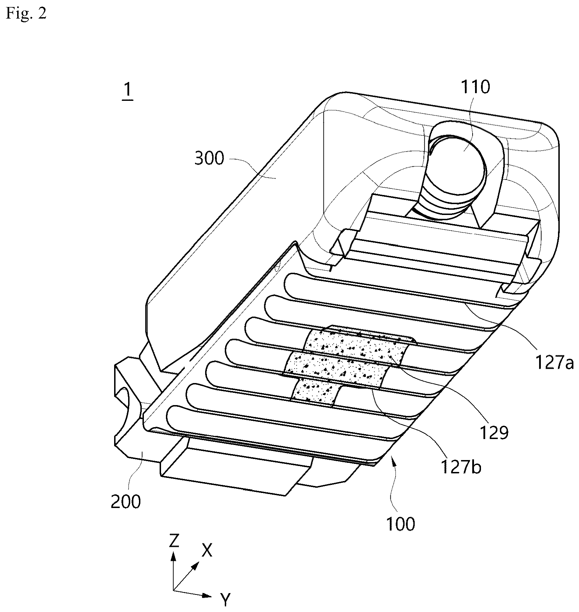

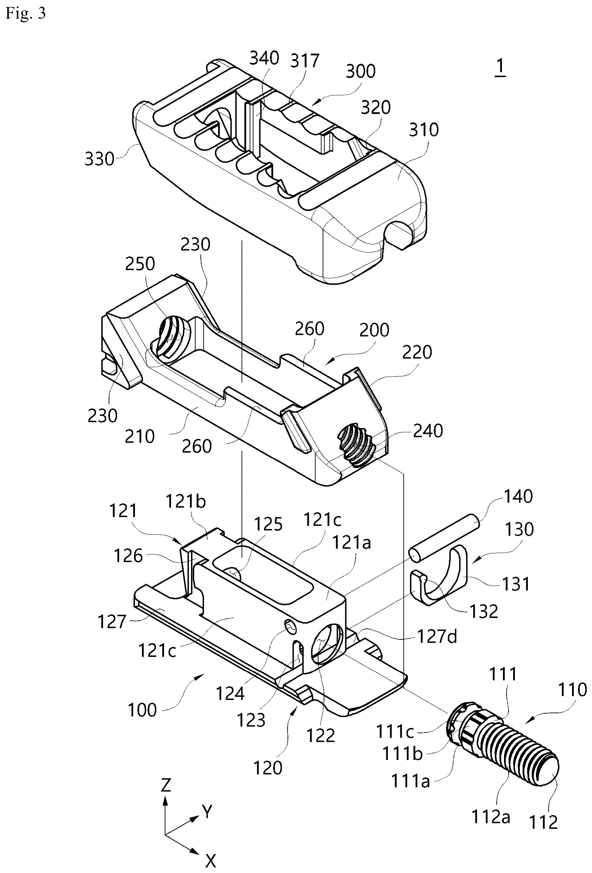

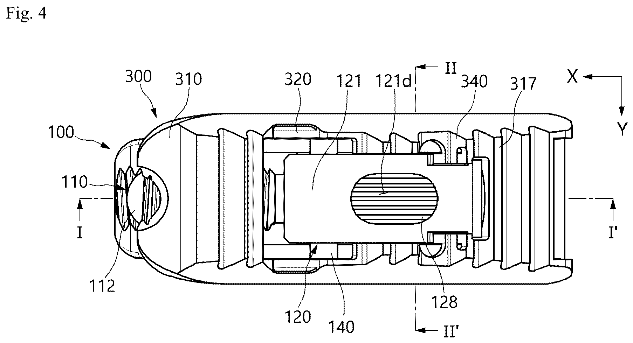

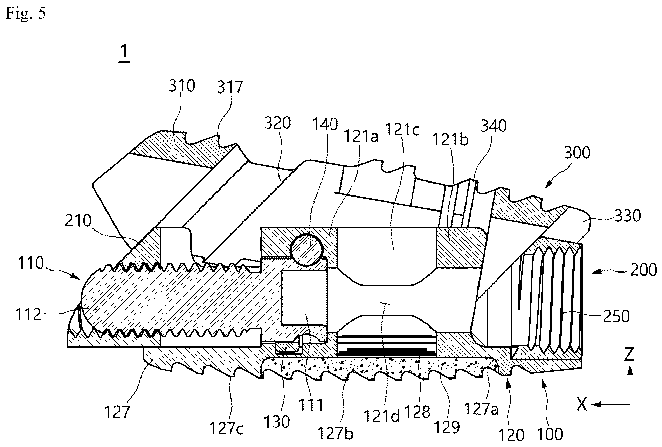

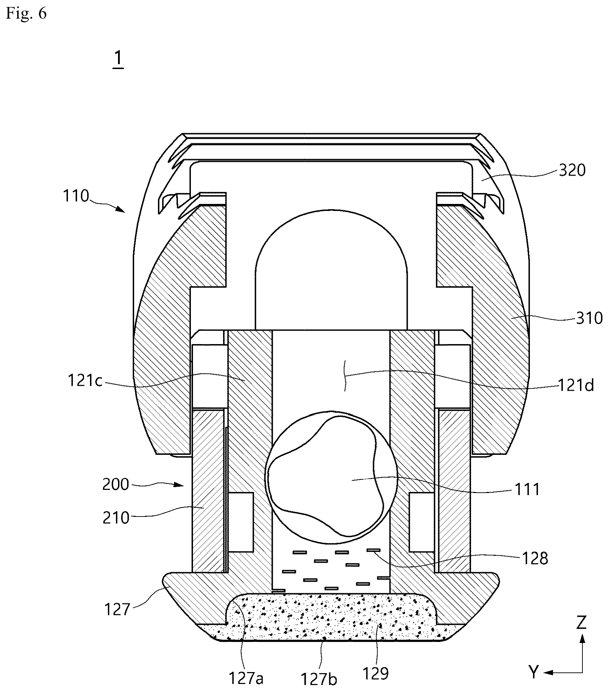

FIG. 1 is a rear perspective view of an intervertebral fusion cage according to an exemplary embodiment of the present invention. FIG. 2 is a bottom perspective view of an intervertebral fusion cage according to an exemplary embodiment of the present invention. FIG. 3 is an exploded perspective view of an intervertebral fusion cage according to an exemplary embodiment of the present invention. FIG. 4 is a top view of an intervertebral fusion cage according to an exemplary embodiment of the present invention. FIG. 5 is a cross-sectional view showing a section of the intervertebral fusion cage taken along line I-I' of FIG. 4. FIG. 6 is a cross-sectional view showing a section of the intervertebral fusion cage taken along line II-II' of FIG. 4.

Referring to FIGS. 1 to 3, an intervertebral fusion cage 1 according to an exemplary embodiment of the present invention may include a main body 100, a guide member 200, and a moving plate 300.

The main body 100 performs relative movement with the guide member 200 according to the rotation of a screw 110 and causes displacement of the moving plate 300. The main body 100 may include a screw 110, a screw housing 120, a torque limiter 130, and a detachment preventing member 140.

The screw 110 includes a screw head 111 and a screw body 112 that extends from the screw head 111 and has a screw thread 112a on its outer circumferential surface.

The screw head 111 has at least one first groove 111a on its outer circumferential surface. The first groove 111a may be formed along the longitudinal direction of the screw 110. In addition, a plurality of first grooves 111a may be arranged at regular intervals along the circumferential direction of the screw head 111. Meanwhile, between adjacent first grooves 111a, a first protrusion 111b protruding radially outward relative to the first groove 111a is formed.

In addition, the screw head 111 may further include a second groove 111c continuously recessed along the circumferential direction. The second groove 111c is formed to allow insertion of the detachment preventing member 140.

The screw body 112 is connected to the screw head 111 and has a screw thread 112a on its outer circumferential surface. The screw thread 112a of the screw body 112 engages with the guide member 200 and transmits the rotational force of the screw 110 to the guide member 200 when the screw 110 rotates. The rotational force transmitted to the guide member 200 is converted into a driving force for linear displacement between the screw housing 120 and the guide member 200.

At this time, the screw 110 may be coupled to the guide member 200 such that the screw body 112 extends from the screw head 111 in a predetermined first direction.

At this time, the first direction may be a forward direction, that is, the positive direction of the x-axis as shown in FIGS. 1 to 3. Hereinafter, in an exemplary embodiment of the present invention, the first direction is defined as the forward direction for the description of the intervertebral fusion cage 1.

The screw housing 120 rotatably accommodates the screw head 111. The screw body 112 of the screw 110 is threadedly engaged with the guide member 200, and when the screw 110 is rotated by a driving tool (not shown) coupled to the screw head 111, the screw housing 120 and the guide member 200 move toward or away from each other.

In the intervertebral fusion cage 1 according to an exemplary embodiment of the present invention, the screw housing 120 may include a housing body 121, a screw head placement portion 122, a torque limiter placement portion 123, a detachment preventing member placement portion 124, a guide hole 125, a first connecting portion 126, and a base plate 127.

The housing body 121 may be formed in a box shape having a through-portion 121d that penetrates along a second direction inclined at a predetermined angle with respect to the first direction. A bone graft material may be filled in the through-portion 121d of the housing body 121.

At this time, the angle formed between the first direction and the second direction may be 90 degrees, and the second direction may be a vertical direction, that is, the z-axis direction as shown in FIGS. 1 to 3.

The second direction is not necessarily limited to being perpendicular to the first direction; however, in the following description of an exemplary embodiment of the present invention, the second direction is defined as the vertical direction for the intervertebral fusion cage 1.

The housing body 121 may have a box shape provided with a through-portion 121d that penetrates in the vertical direction. In this case, the left-right width of the housing body 121 may correspond to the left-right width of the vertically penetrating portion of the guide body 210 of the guide member 200, which will be described later.

The screw head placement portion 122 is formed to penetrate the housing body 121 in the front-rear direction. More specifically, the screw head placement portion 122 is formed to penetrate a front wall 121a of the housing body 121 in the front-rear direction. In a state in which the screw head 111 is placed in the screw head placement portion 122, the screw body 112 may protrude forward from the housing body 121.

Accordingly, the housing body 121 may be provided with a through-portion 121d that penetrates in both the front-rear direction and the vertical direction.

In this case, at least one bar-type member 128 may be formed inside the through-portion 121d so as to extend across the through-portion 121d.

In this case, since a driving tool (not shown) coupled to the screw head 111 must pass through a portion of the through-portion 121d that penetrates the housing body 121 in the front-rear direction to rotate the screw, the bar-type member 128 may be formed at a position where the driving tool (not shown) does not pass.

For example, the bar-type member 128 may be formed at an end of the through-portion 121d on the second-direction side, that is, at an upper end or a lower end of the through-portion 121d.

Referring to FIGS. 4 to 6, in one example, the bar-type member 128 is formed at the lower end of the through-portion 121d; however, it is not limited thereto, and the bar-type member 128 may be formed at the upper end of the through-portion 121d or at both the upper and lower ends thereof.

The bar-type member 128 may be arranged to extend across the through-portion 121d in the first direction, that is, in the front-rear direction.

Accordingly, when an external force acts on the intervertebral fusion cage 1 in the front-rear direction, the bar-type member 128 can enhance the resistance to such an external force.

In addition, since the bar-type member 128 is arranged along the front-rear direction of the spine, it is easy to check bone growth when the intervertebral fusion cage is inserted into the spine and radiographic imaging such as X-ray is performed.

In addition, as the bar-type member 128 is arranged to extend across the through-portion 121d in the front-rear direction, the bar-type member 128 may be oriented in a direction perpendicular to resistance protrusions 127b, 127c, and 317, which will be described later.

Accordingly, the bar-type member 128 reinforces resistance against external forces in the front-rear direction, and the resistance protrusions 127b, 127c, and 317 reinforce resistance against external forces in the left-right direction, so that the bar-type member 128 and the resistance protrusions 127b, 127c, and 317 can complement each other to secure resistance in mutually compensating directions.

In addition, with respect to external forces applied at an oblique angle to the front-rear and left-right directions, the bar-type member 128 and the resistance protrusions 127b, 127c, and 317 can jointly resist such forces, thereby ensuring structural stability against external forces from various directions.

Referring to FIGS. 4 to 6, a plurality of bar-type members 128 may be arranged in parallel.

More specifically, the bar-type members 128 may be spaced apart from each other and arranged at different positions in both the left-right direction and the vertical direction. With such an arrangement structure, the bar-type members 128 can secure additional resistance against external forces applied in the left-right and vertical directions.

For the structural effect of the bar-type members 128 as described above, the bar-type members 128 may be formed of a material having higher rigidity than bone.

In addition, at least two of the bar-type members 128 may be arranged such that at least a portion thereof overlaps when viewed from above.

The bar-type members 128 not only allow the combined structure of the fused bone and the intervertebral fusion cage 1 to secure additional rigidity but also serve to provide a path along which bone can grow during the bone formation process.

In this case, as at least two of the bar-type members 128 are arranged such that at least a portion thereof overlaps when viewed from above, bone growth can be guided in a zigzag direction between the bar-type members 128.

Accordingly, after bone fusion, the bar-type members 128 can be coupled to the bone in a more organic manner rather than in a simple linear configuration. In addition, as the bar-type members 128 are arranged to partially overlap each other, they can cover a larger space and area compared to a case in which they are not overlapped.

The torque limiter placement portion 123 is formed in the housing body 121 so that the torque limiter 130 can be disposed therein. More specifically, the torque limiter placement portion 123 may be formed on the front wall 121a of the housing body 121. In an exemplary embodiment of the present invention, the torque limiter placement portion 123 is formed in a shape that surrounds the screw head placement portion 122 along the circumferential direction. In addition, the torque limiter placement portion 123 has a slot shape penetrating both sides so that the torque limiter 130 can be disposed to penetrate a side surface of the front wall 121a of the housing body 121.

The detachment preventing member placement portion 124 is formed in the housing body 121 so that the detachment preventing member 140 can be disposed therein. In an exemplary embodiment of the present invention, the detachment preventing member placement portion 124 is formed as a through-hole penetrating both sides of the housing body 121. More specifically, the detachment preventing member placement portion 124 penetrates both sides of the front wall 121a of the housing body 121 and is formed such that at least a portion of the detachment preventing member 140 can be inserted into the second groove 111c of the screw head 111.

The guide hole 125 is formed to penetrate a rear wall 121b of the housing body 121 in the front-rear direction so that a driving tool (not shown) for rotating the screw can pass through the vertically penetrating portion of the housing body 121 and reach the screw head 111. Through the guide hole 125, the driving tool can reach the screw head 111.

The first connecting portion 126 is formed in the housing body 121 so that the moving plate 300 can be movably connected in the vertical direction. In an exemplary embodiment of the present invention, the first connecting portion 126 is provided on left and right sidewalls 121c of the housing body 121. The first connecting portion 126 engages with a second connecting portion 340 of the moving plate 300, which will be described later. For example, the first connecting portion 126 may include a groove-shaped structure recessed in the vertical direction.

The base plate 127 may be connected to a surface of the housing body 121 on the second-direction side, that is, to a lower surface of the housing body 121. The base plate 127 may be disposed to face the moving plate 300, which will be described later.

The base plate 127 may extend forward, rearward, and to both lateral sides from the lower side of the housing body 121. Accordingly, the base plate 127 may form a lower surface of the intervertebral fusion cage 1.

Meanwhile, a central portion of the base plate 127 may have a vertically penetrating base hole 127a so as to be in communication with the vertically penetrating through-portion 121d of the housing body 121.

At this time, one or more resistance protrusions 127b and 127c protruding downward from the lower surface of the base plate 127 may be formed on the lower surface of the base plate 127.

The resistance protrusions 127b and 127c protrude downward from the lower surface of the base plate 127 and may extend in a third direction perpendicular to the first direction, that is, in the left-right direction as shown in FIGS. 1 to 6.

Accordingly, the resistance protrusions 127b and 127c may be formed in the shape of a chisel extending in the left-right direction overall, and a plurality of the resistance protrusions 127b and 127c may be arranged in parallel along the front-rear direction on the lower surface of the base plate 127.

At this time, since the base hole 127a penetrating the base plate 127 in the vertical direction is formed in the base plate 127, the resistance protrusions 127b and 127c may be classified into resistance protrusions 127b formed in portions where the base hole 127a is not formed and resistance protrusions 127c formed in portions where the base hole 127a is formed.

In this case, each resistance protrusion 127b formed in a portion where the base hole 127a is not located may be formed to extend in the left-right direction from a base portion adjacent to the lower surface of the base plate 127 to a distal end located at the tip of the resistance protrusion 127b.

In contrast, each resistance protrusion 127c formed in a portion where the base hole 127a is located may have base portions positioned on both sides of the base hole 127a, with distal ends connected to each other across the base hole 127a.

Accordingly, the resistance protrusion 127c formed in the portion where the base hole 127a is located may be formed to partially cover a lower portion of the base hole 127a.

When the intervertebral fusion cage 1 is implanted, the lower surface of the base plate 127 may come into direct contact with the vertebral body. At this time, since the resistance protrusions 127b and 127c extend in the left-right direction and protrude from the lower surface of the base plate 127, the resistance protrusions 127b and 127c can resist displacement of the intervertebral fusion cage 1 in the front-rear direction while the cage is in contact with the vertebral body.

At the same time, when an external force acts on the intervertebral fusion cage 1 in the left-right direction, the resistance protrusions 127b and 127c can also perform a resisting function to prevent damage from the external force.

In this case, since the resistance protrusions 127c are also formed in portions where the base hole 127a is located, the internal space of the base hole 127a can be more stably protected, and the structure can provide more stable resistance to external forces compared to a case in which the resistance protrusions 127b are formed only in portions where the base hole 127a is not located.

Referring to FIGS. 5 and 6, a porous member 129 made of a porous material may be provided in the base hole 127a.

The porous member 129 may be formed of a material and structure capable of promoting the bone formation process.

Although a bone graft material for promoting bone formation may be supplied by being filled into an internal space after installation of the intervertebral fusion cage 1, a separate porous member 129 may be pre-provided in the base hole 127a, which is partitioned in a predetermined shape, so as to correspond to the internal shape of the base hole 127a.

In this case, since the porous member 129 may be provided in advance in a shape corresponding to the internal shape of the base hole 127a before implantation of the intervertebral fusion cage 1, the porous member 129 can be arranged while minimizing dead space.

In addition, because it is unnecessary to separately check after implantation of the intervertebral fusion cage 1 whether the porous member 129 completely fills the inside of the base hole 127a, dead space can be minimized, thereby enabling a simple and reliable spinal fusion procedure.

In addition, the base hole 127a is a region where bone is generated earlier than in the through-portion 121d, and it may also be possible to place a separate bone graft material optimized for initial bone formation in the base hole 127a.

Furthermore, since the porous member 129 of the base hole 127a and the resistance protrusions 127b and 127c can resist external forces together from the initial stage of bone formation, it is possible to achieve more effective resistance to external forces.

Meanwhile, referring to FIGS. 5 and 6, when viewed in a cross section perpendicular to the vertical direction, that is, in a horizontal cross section, the base hole 127a may be formed to have a larger cross-sectional area than an upper end or a lower end of the through-portion 121d where the bar-type member 128 is disposed.

As described above, during the bone formation process, the base hole 127a is positioned closer to the contact surface with the vertebral body than the through-portion 121d, so bone may be generated first in the base hole 127a before bone is generated in the through-portion 121d.

Accordingly, in order to secure structural stability even in the initial stage of bone formation, the horizontal cross-sectional area of the base hole 127a may be formed to be larger than that of the upper or lower end of the through-portion 121d.

As the base hole 127a has a larger horizontal cross-sectional area, initial bone formation can be promoted over a wider area.

After implantation and expansion of the intervertebral fusion cage 1, a separate bone graft material may be filled inside the through-portion 121d, and a relatively large horizontal cross-sectional area may be secured at the central portion of the through-portion 121d in the vertical direction to allow insertion of a driving tool (not shown). Accordingly, the central portion of the through-portion 121d can secure a stable structure against external forces.

In contrast, the upper and lower end portions of the through-portion 121d, where the through-portion 121d is connected to the base hole 127a, may be formed to have a smaller horizontal cross-sectional area than the central portions of the base hole 127a and the through-portion 121d in the vertical direction.

Accordingly, the upper and lower end portions of the through-portion 121d are provided with the bar-type members 128 to reinforce structural stability.

In summary, at the initial stage of bone formation, the base hole 127a having a large horizontal cross-sectional area can promote bone formation over a wide area while supporting the combined structure of the intervertebral fusion cage 1 and the bone.

Subsequently, as bone grows into the upper and lower end portions of the through-portion 121d, which have a relatively small horizontal cross-sectional area, high-rigidity bar-type members 128 provided across the through-portion 121d can secure supporting strength.

Thereafter, as bone grows into the central portion of the through-portion 121d in the vertical direction, which has a relatively large horizontal cross-sectional area, the bone can fuse and be supported within a wide space, allowing the combined structure of the intervertebral fusion cage 1 and the bone to secure a stable structure.

Meanwhile, guide grooves 127d may be provided on both sides of the base plate 127 to guide insertion of the torque limiter 130 into the torque limiter placement portion 123, the guide grooves 127d extending from edges of the base plate 127 toward the torque limiter placement portion 123.

The torque limiter 130 is disposed in the screw housing 120 and prevents rotation of the screw head 111 with respect to torque less than a preset limit torque, while allowing rotation of the screw head 111 when torque equal to or greater than the limit torque is applied. In an exemplary embodiment of the present invention, the torque limiter 130, in a state inserted into the first groove 111a of the screw head 111, restricts rotation of the screw head 111 before the preset limit torque is applied, and when torque equal to or greater than the limit torque is applied, the torque limiter 130 disengages from the first groove 111a due to the adjacent first protrusion 111b, thereby allowing rotation of the screw head 111.

The torque limiter 130 may include a member body 131 that surrounds at least a portion of the circumference of the screw head 111, and a locking portion 132 that protrudes from the member body 131 toward the screw head 111 so as to be inserted into the first groove 111a. For example, the member body 131 may be disposed to surround approximately half of the circumference of the screw head 111 along the circumferential direction, and the locking portion 132 may protrude from one end of the member body 131 toward the screw head 111. In this regard, when torque equal to or greater than the limit torque is applied to the screw 110, the member body 131 may be elastically deformed.

The detachment preventing member 140 is fixedly disposed in the screw housing 120 in a state inserted into a portion of the second groove 111c to prevent the screw 110 from disengaging from the screw housing 120. In an exemplary embodiment of the present invention, the detachment preventing member 140 may be formed in the shape of a pin. More specifically, the detachment preventing member 140 may be inserted into the detachment preventing member placement portion 124, which is formed as a through-hole penetrating both sides of the housing body 121, and at least a portion of the detachment preventing member 140 may be inserted into the second groove 111c of the screw head 111. In addition, the detachment preventing member 140 may be disposed perpendicular to the screw 110.

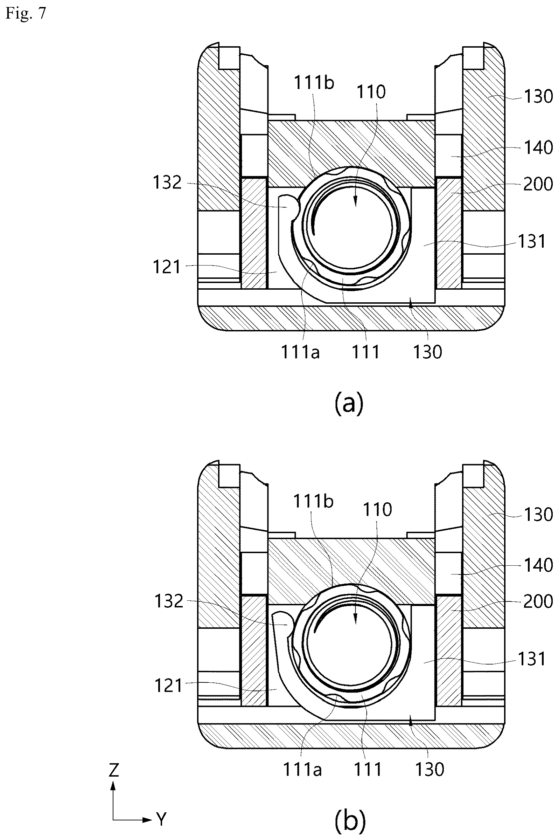

FIG. 7 is a cross-sectional view of an intervertebral fusion cage according to an exemplary embodiment of the present invention, including a section cut in a direction perpendicular to the direction of a screw body at the position of a screw head.

Referring to FIG. 7, in a state where the locking portion 132 of the torque limiter 130 is inserted into the first groove 111a, rotation of the screw 110 is restricted by the locking portion 132 until torque equal to or greater than the limit torque is applied. This is because a protrusion 111b adjacent to the first groove 111a, into which the locking portion 132 is inserted, engages with the locking portion 132 to suppress rotation of the screw.

Meanwhile, when torque equal to or greater than the limit torque is applied to the screw 110, the protrusion 111b of the screw head 111 pushes the locking portion 132 radially outward from the first groove 111a. Accordingly, as the screw head 111 rotates, rotation of the screw 110 occurs. With the rotation of the screw 110, movement of the guide member 200 engaged with the screw 110 may take place.

The guide member 200 is threadedly coupled to the screw body 112 of the screw 110 so as to allow rotational movement of the screw. The guide member 200 performs relative movement with respect to the main body 100, being displaced forward or backward, and displacement of the moving plate 300 occurs according to the relative positional change between the guide member 200 and the main body 100. The guide member 200 may include a guide body 210, a front guide unit 220, a rear guide unit 230, a screw hole 240, and a rear hole 250.

The guide body 210 is formed to be penetrated vertically. The guide body 210 may have a frame shape with a central portion that is penetrated. In addition, the guide body 210 may have an overall rectangular shape. The housing body 121 of the screw housing 120 of the main body 100 is disposed inside the guide body 210, and the screw housing 120 performs relative movement with the guide member 200 inside the guide body 210 according to the rotation of the screw 110 rotatably coupled to the screw housing 120. As described above, the vertically penetrating portion of the guide body 210 may have a left-right width corresponding to the left-right width of the housing body 121 of the main body 100.

The front guide unit 220 is formed to be inclined at a front portion of the guide body 210. The front guide unit 220 guides a front guided unit 320 of the moving plate 300 during displacement of the guide member 200 and guides the upward movement of a front portion of the moving plate 300. In an exemplary embodiment of the present invention, the front guide unit 220 protrudes from both sides of a sidewall at the front portion of the guide body 210 and is formed to be inclined downward at a predetermined angle from the rear side toward the front side.

The rear guide unit 230 is formed to be inclined at a rear portion of the guide body 210. The rear guide unit 230 guides a rear guided unit 330 of the moving plate 300 during displacement of the guide member 200. In other words, the rear guide unit 230 guides the upward movement of a rear portion of the moving plate 300. In an exemplary embodiment of the present invention, the rear guide unit 230 protrudes from both sides of a sidewall at the rear portion of the guide body 210 and is formed to be inclined downward at a predetermined angle from the rear side toward the front side.

A screw hole 240 may be formed at the front portion of the guide body 210. The screw hole 240 may be formed to penetrate the front sidewall of the guide body 210 in the front-rear direction. The screw body 112 of the screw 110 of the main body 100 disposed in the guide body 210 may be engaged and positioned in the screw hole 240. Accordingly, when the screw 110 is driven, the guide member 200 can perform relative movement forward or backward with respect to the screw housing 120 of the main body 100.

A rear hole 250 is formed at the rear of the guide body 210 so as to be in communication with the guide body 210. More specifically, the rear hole 250 may be formed to penetrate the rear sidewall of the guide body 210 in the front-rear direction. The rear hole 250 allows a driving tool for driving the screw 110 of the main body 100 to enter the interior of the guide body 210. The driving tool that has entered the guide body 210 through the rear hole 250 can reach the screw 110 by passing through the guide hole 125 of the screw housing 120.

The moving plate 300 is movably connected to the screw housing 120 and is displaced according to relative movement between the main body 100 and the guide member 200. When the screw 110 rotates, the main body 100 performs relative movement forward or backward with respect to the guide member 200, and the moving plate 300 may be displaced by the guide member 200. Here, the displacement of the moving plate 300 may refer to movement of a portion or the entirety of the moving plate 300 upward or downward.

The moving plate 300 can define the lordosis and height of the intervertebral fusion cage 1. In an exemplary embodiment of the present invention, the moving plate 300 forms an upper surface of the intervertebral fusion cage 1. The moving plate 300 may include a plate body 310, a front guided unit 320, a rear guided unit 330, and a second connecting portion 340.

The plate body 310 has an overall rectangular shape and is penetrated in the vertical direction. The plate body 310 has sidewalls extending downward on all sides with respect to the penetrated portion.

At this time, one or more resistance protrusions 317 protruding upward from the upper surface of the moving plate 300 may be formed on the upper surface of the plate body 310.

The resistance protrusions 317 protrude upward from the upper surface of the plate body 310 and may extend in a third direction perpendicular to the first direction, that is, in the left-right direction as shown in FIGS. 1 to 6.

Accordingly, the resistance protrusions 317 may be formed in the shape of a chisel extending in the left-right direction overall, and a plurality of the resistance protrusions 317 may be arranged in parallel along the front-rear direction on the upper surface of the plate body 310.

The front guided unit 320 is guided by the front guide unit 220 of the guide member 200. When the moving plate 300 performs relative movement with respect to the guide member 200 and the front guided unit 320 is guided by the front guide unit 220, a front portion of the moving plate 300 may be raised. In an exemplary embodiment of the present invention, the front guided unit 320 is formed as a recessed portion on inner surfaces of both sidewalls of the plate body 310 and is recessed in a downwardly inclined shape from the rear side toward the front side.

The rear guided unit 330 is guided by the rear guide unit 230 of the guide member 200. When the moving plate 300 performs relative movement with respect to the guide member 200 and the rear guided unit 330 is guided by the rear guide unit 230, a rear portion of the moving plate 300 may be raised. In an exemplary embodiment of the present invention, the rear guided unit 330 is provided on a rear sidewall of the plate body 310 and is formed to be inclined downward from the rear side toward the front side.

The second connecting portion 340 is movably connected to the first connecting portion 126 in the vertical direction. The second connecting portion 340 connects the moving plate 300 to the screw housing 120 of the main body 100. In an exemplary embodiment of the present invention, the second connecting portion 340 may be movably connected in the vertical direction to the first connecting portion 126 of the screw housing 120. In an exemplary embodiment of the present invention, the second connecting portion 340 may protrude from inner surfaces of both sidewalls of the plate body 310 and may include a portion extending in the vertical direction.

Hereinafter, the operation of the intervertebral fusion cage 1 according to an exemplary embodiment of the present invention will be described.

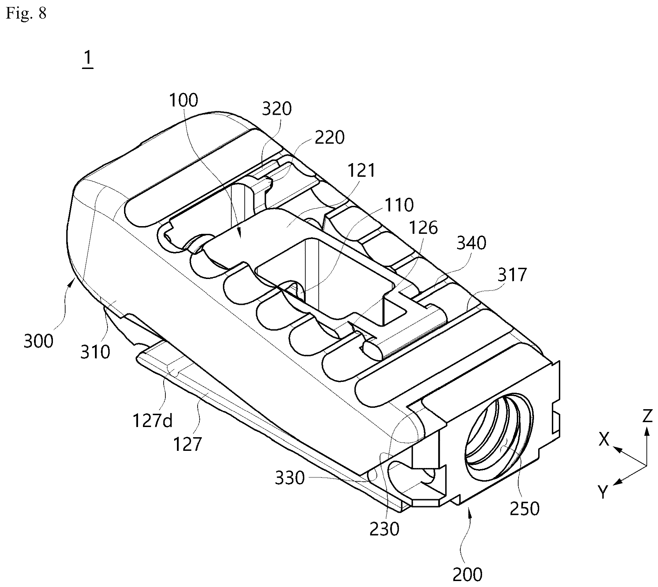

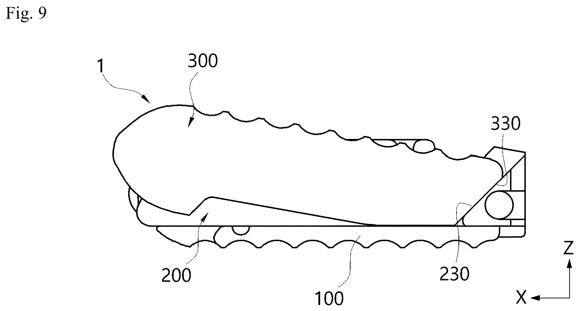

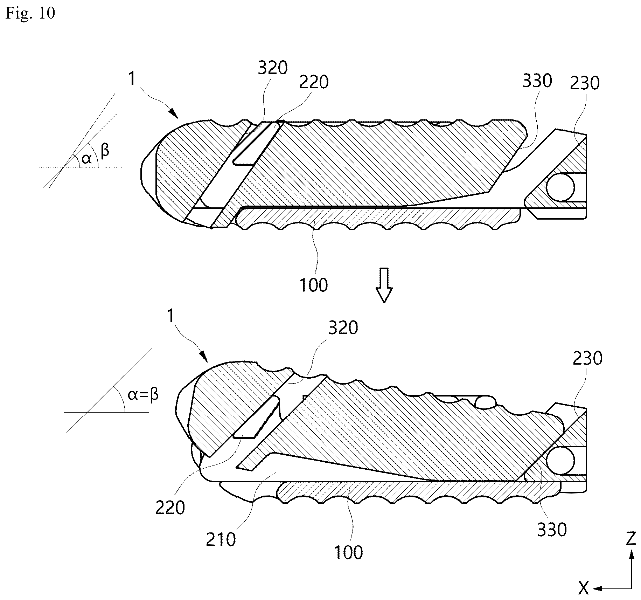

FIG. 8 is a rear perspective view showing an intervertebral fusion cage in an expanded state of lordosis according to an exemplary embodiment of the present invention. FIG. 9 is a left side view of the intervertebral fusion cage shown in FIG. 8. FIG. 10 is a side cross-sectional view illustrating a process in which an angle between a front guide unit and a front guided unit of the intervertebral fusion cage according to an exemplary embodiment of the present invention is aligned.

Referring to FIGS. 8 to 10, when the screw 110 of the main body 100 rotates in one direction in a state where the moving plate 300 is not expanded with respect to the guide member 200, the guide member 200 moves forward relative to the main body 100, causing displacement of the moving plate 300. At this time, a front portion of the moving plate 300 first moves in the vertical direction, thereby increasing the angle formed between the moving plate 300 and the guide member 200. In this regard, the rotation of the screw 110 may occur when torque equal to or greater than the limit torque is applied to the screw 110.

As shown in FIG. 10, the angular expansion of the front portion of the moving plate 300 proceeds until an angle ( α) formed between the front guided unit 320 of the moving plate 300 and the ground becomes equal to an angle ( β) formed between the front guide unit 220 of the guide member 200 and the ground. In the non-expanded state of the moving plate 300, the angle ( β) formed between the front guide unit 220 and the ground is smaller than the angle ( α) formed between the front guided unit 320 and the ground.

Accordingly, during displacement of the moving plate 300, the front portion of the moving plate 300 rises until the angle formed between the front guide unit 220 and the front guided unit 320 with respect to the ground becomes equal, thereby causing expansion of the lordosis. In other words, the angle ( β) formed between the front guide unit 220 and the ground may correspond to the spinal lordosis that the intervertebral fusion cage 1 can provide. Meanwhile, as the front portion of the moving plate 300 rises, the moving plate 300 moves rearward relative to the guide member 200, causing the rear guided unit 330 of the moving plate 300 to come into contact with the rear guide unit 230 of the guide member 200.

As described above, in the initial stage of displacement of the moving plate 300, the front portion of the moving plate 300 rises until the angle formed between the front guide unit 220 and the ground becomes equal to the angle formed between the front guided unit 320 and the ground. As a result, expansion of the lordosis may occur. Through this process, the intervertebral fusion cage 1 can form the spinal lordosis at a predetermined angle while being inserted in the intervertebral space of the patient.

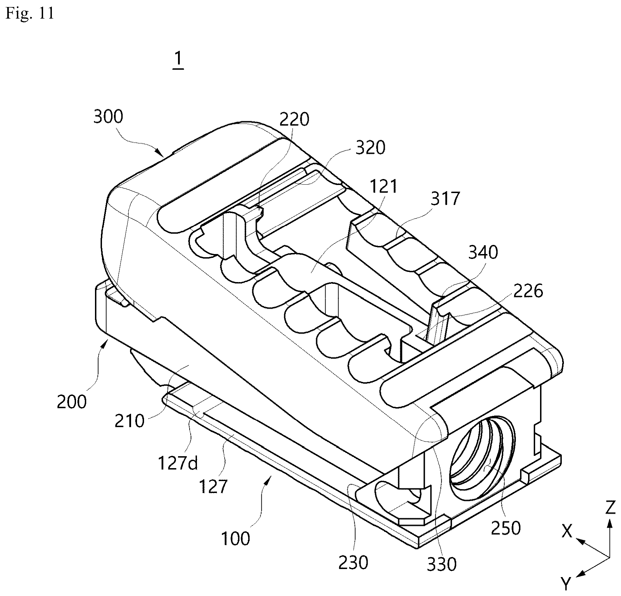

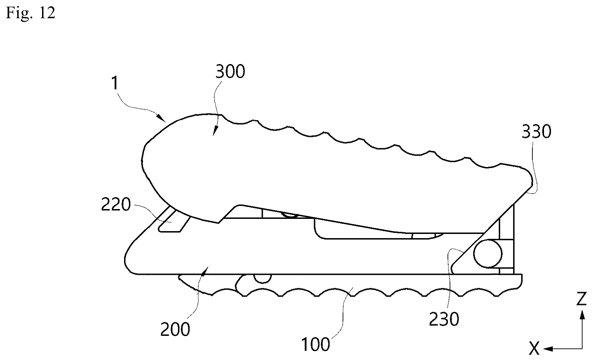

FIG. 11 is a perspective view showing an intervertebral fusion cage in a state in which the height of a plate is increased after expansion of the lordosis according to an exemplary embodiment of the present invention. FIG. 12 is a left side view of the intervertebral fusion cage shown in FIG. 11.

Referring to FIGS. 11 and 12, when the front portion of the moving plate 300 is maximally raised so that the angle formed between the front guided unit 320 of the moving plate 300 and the ground becomes equal to the angle formed between the front guide unit 220 of the guide member 200 and the ground, and when the rear guided unit 330 of the moving plate 300 comes into contact with the rear guide unit 230 of the guide member 200, additional rotation of the screw 110 of the main body 100 in one direction causes the entire moving plate 300 to move in the vertical direction, thereby increasing its height.

That is, after the angle increases by the predetermined set angle, when the screw 110 is further rotated, the front guided unit 320 and the rear guided unit 330 of the moving plate 300 come into contact with the front guide unit 220 and the rear guide unit 230 of the guide member 200, respectively, and the guide member 200 moves forward relative to the main body 100, causing the entire moving plate 300 to move vertically upward. Accordingly, the height of the intervertebral fusion cage 1 is expanded. At this time, the expansion height may be set to correspond to the height of the intervertebral space that the intervertebral fusion cage 1 is intended to secure.

In this regard, the rotation of the screw 110 may occur when torque equal to or greater than the limit torque is applied to the screw 110.

The displacement of the intervertebral fusion cage 1 according to an exemplary embodiment of the present invention is reversible. In other words, when torque equal to or greater than the limit torque is applied to the screw 110 in the other direction while the intervertebral fusion cage 1 is in an expanded state, the displacement of the guide member 200 and the moving plate 300 described above can proceed in reverse.

Meanwhile, in connection with the relative movement between the main body 100 and the guide member 200, the guide member 200 may further include lateral guide units 260 formed in the front-rear direction on both sidewalls of the guide body 210. In addition, the detachment preventing member 140 is disposed to penetrate both sides of the screw housing 120, and its one end and the other end may be seated on the lateral guide units 260, respectively.

In an exemplary embodiment of the present invention, the lateral guide units 260 may be provided at upper edge portions of both sidewalls of the guide body 210. In addition, one end and the other end of the pin-shaped detachment preventing member 140 may protrude to both sides of the screw housing 120 and be guided by the lateral guide units 260 when the main body 100 moves relative to the guide member 200.

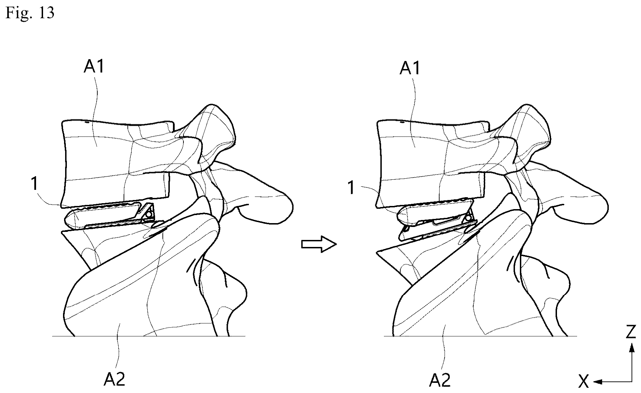

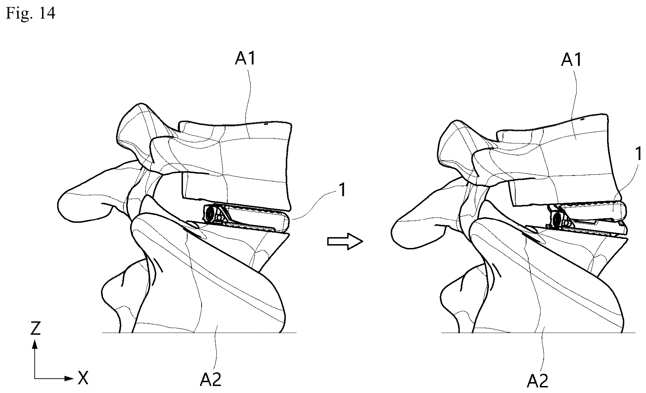

FIG. 13 is a view illustrating a process in which the intervertebral fusion cage according to an exemplary embodiment of the present invention is used in a PLIF (Posterior Lumbar Interbody Fusion). FIG. 14 is a view illustrating a process in which the intervertebral fusion cage according to an exemplary embodiment of the present invention is used in a TLIF (Transforaminal Lumbar Interbody Fusion).

Referring to FIGS. 13 and 14, the intervertebral fusion cage 1 according to an exemplary embodiment of the present invention can be used for both PLIF (Posterior Lumbar Interbody Fusion) and TLIF (Transforaminal Lumbar Interbody Fusion). The intervertebral fusion cage 1 is inserted into a space between an upper vertebral body A1 and a lower vertebral body A2, allowing both the lordosis and the height to be expanded. In addition, since the cage expands after being inserted into the patient's body, it enables a minimally invasive procedure during implantation.

Although exemplary embodiments of the present invention have been described above, the scope of the present invention is not limited to the embodiments set forth herein. Those skilled in the art who understand the spirit of the present invention may readily propose other embodiments by adding, modifying, deleting, or supplementing components within the scope of the present invention, and such embodiments should also be regarded as falling within the scope of the present invention.

## Claims

1. An intervertebral fusion cage, comprising:
a screw including a screw head and a screw body which extends from the screw head in a first direction and has a screw thread on an outer circumferential surface of the screw body;
a main body including a screw housing in which the screw head is rotatably accommodated and which has a through-portion penetrating in a second direction inclined with respect to the first direction, and a base plate which is disposed on one surface of the screw housing on the second-direction side and has a base hole in communication with the through-portion;
a guide member including a guide body in which the screw housing is accommodated and to which the screw body is rotatably coupled, the guide member being movable relative to the main body in a direction parallel to the first direction according to rotation of the screw;
a moving plate movably connected to the main body and displaced according to relative movement between the main body and the guide member; and
at least one bar-type member disposed across the through-portion.

2. The intervertebral fusion cage of claim 1, wherein the bar-type member is disposed at an end of the through-portion on the second-direction side.

3. The intervertebral fusion cage of claim 3, wherein the bar-type member is disposed to extend across the through-portion along the first direction.

4. The intervertebral fusion cage of claim 1, wherein a plurality of the bar-type members are arranged in parallel with each other.

5. The intervertebral fusion cage of claim 1, wherein a plurality of the bar-type members are spaced apart from each other and disposed at positions having different heights.

6. The intervertebral fusion cage of claim 1, wherein a plurality of the bar-type members are provided, and at least two of the bar-type members are disposed such that at least a portion thereof overlap with each other when viewed in the second direction.

7. The intervertebral fusion cage of claim 1,
wherein the base plate comprises at least one resistance protrusion formed to protrude on the one surface on the second-direction side,
and the resistance protrusion extends in a third direction perpendicular to the first direction.

8. The intervertebral fusion cage of claim 7,
wherein a plurality of the resistance protrusions are formed,
at least one of the resistance protrusions is formed on a portion of the one surface of the base plate on the second-direction side where the base hole is not located,
and at least one of the resistance protrusions is formed such that an end portion of the resistance protrusion extends across the base hole along the third direction.

9. The intervertebral fusion cage of claim 3, wherein a porous member made of a porous material is provided in the base hole.

10. The intervertebral fusion cage of claim 9, wherein the porous member is provided in a shape corresponding to the shape of the base hole.

11. The intervertebral fusion cage of claim 1,
wherein the second direction is formed at an angle perpendicular to the first direction,
and an area of a cross section obtained by cutting the base hole in a direction perpendicular to the second direction is larger than an area of a cross section obtained by cutting an end portion of the through-portion on the second-direction side in the direction perpendicular to the second direction.

12. The intervertebral fusion cage of claim 1,
wherein the moving plate comprises at least one upper resistance protrusion formed to protrude on one surface thereof farthest from the base plate,
and the upper resistance protrusion extends in a third direction perpendicular to the first direction.
